# EUROPEAN PATENT APPLICATION

(11) **EP 4 035 672 A1**
(43) Date of publication of application: **03.08.2022**
(21) Application number: 20878112.0
(22) Date of filing: 20.10.2020
(51) Int. Cl.: A61K 38/47, A61K 31/7048, A61P 1/04

(54) **DRUG COMBINATION FOR PREVENTING OR TREATING IRRITABLE BOWEL SYNDROME**

(30) Priority: 25.10.2019 CN 201911025557
(71) Applicant: Guangzhou Century Clinical Research Co., Ltd, Guangzhou, Guangdong 510530 (CN); Guangzhou Xin-Chuangyi Biopharmaceutical Co., Ltd, Huangpu District Guangzhou, Guangdong 510535 (CN); Xiangbei Welman Pharmaceutical Co., Ltd, Hunan 410331 (CN); Nanjing Kangfushun Pharmaceutical Co., Ltd, Nanjing, Jiangsu 210046 (CN)
(72) Inventor: SUN, Mingjie, Guangzhou, Guangdong 510620 (CN); SUN, Tianyu, Guangzhou, Guangdong 510620 (CN); JIANG, Juanyan, Guangzhou, Guangdong 510620 (CN)
(74) Representative: Ladendorf, Oliver
(86) International application number: PCT/CN2020/122082
(87) International publication number: WO 2021/078110

(57) **Abstract**

Disclosed is a drug combination for preventing or treating irritable bowel syndrome. The drug combination comprises dosmalfate and lysozyme.

## Description

### TECHNICAL FIELD

The present disclosure relates to the field of medicines, particularly relates to a drug combination for preventing or treating irritable bowel syndrome.

### BACKGROUND

Irritable bowel syndrome (IBS) is a common functional intestinal disease with high morbidity. Meta analysis shows that Chinese people have an IBS morbidity of 1.0%-16.0% and a total morbidity of 6.5%, among them, a morbidity meeting Manning standard is 11.5% and a morbidity meeting Roma III standard is 8.9%.

IBS is a chronic disease characterized by abdominal pain or discomfort associated with changes in defecation or defecation habit change relevant abdominal pain or discomfort. Abdominal pain, bloating, distension and dysporia are common symptoms of IBS and can repeatedly occur, so as to bring great pains to patients and reduce the quality of life.

According to Roma III standard and based on stool properties of patients, IBS can be divided into diarrhea irritable bowel syndrome (IBS-D), constipation irritable bowel syndrome (IBS-C), mixed irritable bowel syndrome (IBS-M) and Un-subtyped IBS. Patients usually switch among these subtypes.

IBS lacks detectable organic etiology, and its etiology remains unknown. Laboratory examination, X-ray examination and biopsy show no anatomical abnormalities. Emotional factors, diet, drugs or hormones may trigger or exacerbate gastrointestinal symptoms.

Visceral hypersensitivity is a core pathogenesis of IBS, which plays an important role in symptom occurrence and disease development of IBS. Most of researches prove that patients with IBS have visceral hypersensitivity to rectum and colon, that is, perceptibility of intestinal tracts on stimulation is enhanced. The visceral hypersensitivity is a core pathophysiological mechanism of abdominal discomfort symptoms such as abdominal pain and abdominal bloating in irritable bowel syndrome. Patients with IBS are sensitive to colorectal distension (pressure) stimulation, and the pain thresholds of patients with irritable bowel syndromes, whether diarrhea subtype or constipation subtype, are lower than those of control groups.

The current drugs for treating irritable bowel syndrome include antispasmodic drugs, laxatives, antidiarrheal, antidepressants (TCAs, SSRIs), antibiotics, 5-HT3 receptor antagonists, 5-HT4 receptor partial agonists, guanylate cyclase-C agonists and µ-opioid receptor agonists, but the existing drugs for treating irritable bowel syndrome have obvious side effects. Antispasmodic drugs can alleviate the symptoms of patients with diarrhea irritable bowel syndrome in a short time, and have a certain curative effect on abdominal pain. Such drugs include selective intestinal smooth muscle relaxants (such as pinaverium bromide, otilonium bromide, cimetropium bromide, mebeverine and alverine) and anticholinergic drugs (such as scopolamine), etc. The common side effects of smooth muscle relaxation antispasmodic drugs include dyspnea, headache, dizziness, skin pruritus and rash, etc. Anticholinergic antispasmodic drugs can cause quickened heartbeat, blurred vision, constipation, etc., thus should be used with caution by patients with gastroesophageal reflux disease, acute myocardial infarction, hypertension, hyperthyroidism and patients prone to angle closure glaucoma, and should be avoided by patients with benign prostatic hyperplasia. Alosetron, a 5-HT3 receptor antagonist, is initially approved by FDA in the United States to treat female patients with diarrhea irritable bowel syndrome. This drug can cause ischemic colitis and constipation complications, so the scope of its indications is narrowed and it is only used in patients with severe symptoms. Tegaserod, a 5-HT4 receptor partial agonist, is approved for short-term treatment for symptomatic relief in female patients with constipation irritable bowel syndrome. However, this drug has been suspended to be sold in the United States due to the risk of adverse cardiovascular events. Rifaximin is approved for treating diarrhea irritable bowel syndrome in the United States, but there is a black box warning about *Clostridium difficile* related diarrhea in the label of the instruction.

There is still an urgent need for drugs for treating irritable bowel syndrome with a good curative effect, less adverse reactions or less side effects.

### SUMMARY

The objective of the present disclosure is to provide a use of a drug combination of dosmalfate and lysozyme in preparation of a medicament for preventing or treating irritable bowel syndrome, a pharmaceutical composition for preventing or treating irritable bowel syndrome, and a method of preventing or treating irritable bowel syndrome.

Based on this, in the first aspect of the present disclosure , provided is a use of a drug combination in preparation of a medicament for preventing or treating irritable bowel syndrome, the drug combination comprising dosmalfate and lysozyme. The inventors find that combined use of dosmalfate and lysozyme can prevent and treat irritable bowel syndrome, the combination of the two has a significant synergistic effect.

In some embodiments, the irritable bowel syndrome has the characteristic of visceral hypersensitivity.

The inventors also find that a mass ratio of dosmalfate to lysozyme in the drug combination has a great effect on their synergistic treatment of irritable bowel syndrome. In some embodiments, the mass ratio of dosmalfate to lysozyme in the drug combination is (0.05-100) :1, and does not comprise 0.05:1. In some embodiments, the mass ratio of dosmalfate to lysozyme in the drug combination is (0.1-10) :1. When the mass ratio of dosmalfate to lysozyme is 0.05:1, there is no synergistic effect; when the mass ratio of dosmalfate to lysozyme is (0.1-10):1, there is a significant synergistic effect; when the ratio exceeds this interval, the synergistic effect is weakened.

In some embodiments, the potency of the lysozyme is more than 20000 U/mg.

The dosmalfate and lysozyme in the drug combination can be respectively administrated (combined medication) in a form of separate formulations, or in a form of existing in the same formulation (pharmaceutical composition). In some embodiments, the dosmalfate and lysozyme are administrated in a form of pharmaceutical composition.

In some embodiments, the pharmaceutical composition is an oral formulation. In some embodiments, the oral formulation is any one selected from an oral normal-release formulation, an oral sustained-release formulation, and an oral controlled-release formulation. In some embodiments, the oral formulation is any one selected from a tablet, a capsule, a granule, powder, a pill, syrup, an oral solution, an oral suspension and an oral emulsion.

In some embodiments, the pharmaceutical composition also comprises pharmaceutically acceptable adjuvants. In some embodiments, the pharmaceutically acceptable adjuvants are at least one selected from the group consisting of fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

In some embodiments, the dosmalfate and lysozyme are respectively administrated in a form of separate formulations.

In some embodiments, the separate formulation also comprises pharmaceutically acceptable adjuvants. In some embodiments, the pharmaceutically acceptable adjuvants are at least one selected from the group consisting of fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

By further researches, the inventors find that compared with other formulations of lysozyme, when the lysozyme is prepared into an enteric formulation, especially a formulation that releases the drug in large intestine, especially in colon, it can exert a better synergistic effect with dosmalfate so as to produce a better curative effect when treating IBS.

According to concrete needs, the enteric formulation is in an enteric formulation form with an enteric function, such as a normal-release formulation with enteric function, a sustained-release formulation with enteric function and a controlled-release formulation with enteric function, and can be in an enteric formulation form with an enteric function, such as a tablet with enteric function, a capsule with enteric function, a pill with enteric function, a granule with enteric function, or also can be any combinations thereof. In some embodiments, the enteric formulation is an enteric tablet. In some embodiments, the enteric formulation is a colon enteric tablet.

In some embodiments, the dosmalfate is in an oral formulation form. According to concrete needs, the dosmalfate is in a formulation form such as a normal-release formulation, a sustained-release formulation or a controlled-release formulation, and can be of a formulation form such as a tablet, a capsule, a granule, powder, a pill, syrup, an oral solution, an oral suspension and an oral emulsion, or also can be any combinations thereof. In some embodiments, the dosmalfate is the tablet.

In the second aspect of the present disclosure, provided is a pharmaceutical composition for preventing or treating irritable bowel syndrome, the pharmaceutical composition comprising dosmalfate and lysozyme. The inventors find that combined use of dosmalfate and lysozyme can prevent and treat irritable bowel syndrome, and the combination of the two has a significant synergistic effect.

The inventors find that a mass ratio of dosmalfate to lysozyme in the pharmaceutical composition has a great effect on their synergistic treatment of irritable bowel syndrome. In some embodiments, the mass ratio of dosmalfate to lysozyme in the pharmaceutical composition is (0.05-100):1, and does not comprise 0.05:1. In some embodiments, the mass ratio of dosmalfate to lysozyme in the pharmaceutical composition is (0.1-10):1.

In some embodiments, the potency of the lysozyme is more than 20000 U/mg.

In some embodiments, the pharmaceutical composition is an oral formulation. In some embodiments, the oral formulation is any one selected from an oral normal-release formulation, an oral sustained-release formulation and an oral controlled-release formulation. In some embodiments, the oral formulation is any one selected from a tablet, a capsule, a granule, powder, a pill, syrup, an oral solution, an oral suspension and an oral emulsion.

In some embodiments, the pharmaceutical composition also comprises pharmaceutically acceptable adjuvants. In some embodiments, the pharmaceutically acceptable adjuvants are at least one selected from the group consisting of fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

The pharmaceutical composition can be prepared by the following methods:
The pharmaceutical composition can be prepared by mixing dosmalfate with lysozyme and then adding pharmaceutically acceptable adjuvants.

Alternatively, the pharmaceutical composition can be obtained by respectively preparing a dosmalfate formulation subunit and a lysozyme formulation subunit with dosmalfate and lysozyme as well as the pharmaceutically acceptable adjuvants, and mixing the two formulation subunits. Preferably, the dosmalfate formulation subunit is in an enteric dosage form or gastric dosage form, and the lysozyme formulation subunit is in the enteric dosage form.

In the third aspect of the present disclosure, provided is a method of preventing or treating irritable bowel syndrome, comprising: administrating an effective amount of dosmalfate and lysozyme to patients with IBS.

The inventors find that the mass ratio of dosmalfate to lysozyme has a great effect on their synergistic treatment of irritable bowel syndrome. In some embodiments, the mass ratio of dosmalfate to lysozyme is (0.05-100) :1, and does not comprise 0.05:1. In some embodiments, the mass ratio of dosmalfate to lysozyme in the drug combination is (0.1-10):1.

In some embodiments, the potency of the lysozyme is more than 20000 U/mg.

In some embodiments, the daily dosage of the dosmalfate is 0.05-20 g; preferably, the daily dosage of the dosmalfate is 0.1-10 g.

In some embodiments, the dosmalfate or lysozyme can be respectively administrated for 1-3 times/day.

In some embodiments, the dosmalfate or lysozyme can be respectively administrated (combined medication) in a form of separate formulations, or in a form of existing in the same formulation (pharmaceutical composition).

In some embodiments, the dosmalfate and lysozyme are administrated in a form of pharmaceutical composition.

In some embodiments, the pharmaceutical composition is an oral formulation. In some embodiments, the oral formulation is any one selected from an oral normal-release formulation, an oral sustained-release formulation and an oral controlled-release formulation. In some embodiments, the oral formulation is any one selected from a tablet, a capsule, a granule, powder, a pill, syrup, an oral solution, an oral suspension and an oral emulsion.

In some embodiments, the pharmaceutical composition also comprises pharmaceutically acceptable adjuvants. In some embodiments, the pharmaceutically acceptable adjuvants are at least one selected from the group consisting of fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

In some embodiments, the dosmalfate and lysozyme are respectively administrated in a form of separate formulations.

In some embodiments, the separate formulation also comprises pharmaceutically acceptable adjuvants. In some embodiments, the pharmaceutically acceptable adjuvants are at least one selected from the group consisting of fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

By further researches, the inventors find that compared with other formulations of lysozyme, when the lysozyme is preapred into an enteric formulation, especially a formulation that releases the drug in large intestine, especially in colon, it can exert a better synergistic effect with dosmalfate so as to produce a better curative effect when treating IBS.

According to specific needs, the enteric formulation is in an enteric formulation form with an enteric function, such as a normal-release formulation with enteric function, a sustained-release formulation with enteric function and a controlled-release formulation with enteric function, and can be in an enteric formulation form with an enteric function, such as a tablet with enteric function, a capsule with enteric function, a pill with enteric function, a granule with enteric function, or also can be any combinations thereof. In some embodiments, the enteric formulation is an enteric tablet. In some embodiments, the enteric formulation is a colon enteric tablet.

In some embodiments, the dosmalfate is in an oral formulation form. According to specific needs, the dosmalfate is in a formulation form such as a normal-release formulation, a sustained-release formulation or a controlled-release formulation, and can be in a formulation form such as a tablet, a capsule, a granule, powder, a pill, syrup, an oral solution, an oral suspension and an oral emulsion, or any combinations thereof.

In some embodiments, the dosmalfate is a tablet.

The present disclosure has the beneficial effects:
The combination of dosmalfate and lysozyme provided by the present disclosure can significantly improve irritable bowel syndrome, and the combination of the two has a synergistic effect in improving irritable bowel syndrome. Through the test, it is found in the present disclosure that the combination of dosmalfate and lysozyme can significantly reduce abdominal wall withdrawal reflex (AWR) scores and electromyography (EMG) AUC values of irritable bowel syndrome model animals, and obviously improve the visceral high sensitivity state. In addition, since visceral hypersensitivity features exist in various types of irritable bowel syndromes, the combination of the present disclosure can be applied to various types of irritable bowel syndromes. Moreover, none of all test animals shows significant toxicity after being continuously administrated for one month, indicating fewer side effects. Therefore, the present disclosure provides a new drug combination with wide application, good curative effect and fewer side effects for treating irritable bowel syndrome.

### DETAILED DESCRIPTION

The following examples are further enumerated to illustrate the present disclosure in detail. It should also be understood that the following examples are only used to further illustrate the present invention, and should not be construed as limiting the protection scope of the present disclosure. Some non-essential improvements and adjustments made by those skilled in the art according to the principles set forth in the present disclosure belong to the scope of the present disclosure. The specific process parameters and the like in the following examples are only an example in the appropriate range, that is, those skilled in the art can make selections within the appropriate range through the descriptions herein, and are not limited to the specific data in the following examples.

Definition:
Irritable bowel syndrome (IBS): IBS is a functional intestinal disease, exhibiting abdominal pain or discomfort associated with changes in defecation or defecation habit change relevant abdominal pain or discomfort. Abdominal pain, bloating, distension and dysporia are common features.

Visceral hypersensitivity: visceral hypersensitivity refers to enhancement of perceptibility of intestinal tract to stimulation, including a reduced threshold triggering visceral pain or uncomfortable stimulation, visceral discomfort to physiological stimulation or a strong response to noxious stimulation. Not only can rectum and colon show visceral hypersensitivity but also other areas of the digestive tract such as jejunum and esophagus can also exhibit visceral hypersensitivity. Animals or patients with irritable bowel syndrome have visceral hypersensitivity. Visceral hypersensitivity is a core pathophysiological mechanism of abdominal discomfort symptoms such as abdominal pain and abdominal bloating in IBS. Visceral hypersensitivity can amplify gastrointestinal motility events to produce symptoms. Visceral hypersensitivity in animals or patients with irritable bowel syndrome includes, but is not limited to, sensitivity to colorectal distention (pressure) stimulation, high sensitivity to temperature stimulation, and high responsiveness to physiological stimulation (such as meals).

Dosmalfate: dosmalfate is a gastric mucosa protecting agent approved for treatment of gastric and duodenal ulcers. Its chemical name is a diosmin heptadiene (bisulfate) aluminum complex with a molecular formula Al₇(OH)₁₄(C₂₈H₂₅O₃₆S₇)[Al(OH)₃]₇. Animal experiment and human body researches both show that dosmalfate can effectively treat peptic ulcer, and its adverse reactions are mild and the incidence is low.

Lysozyme: lysozyme is a lysozyme derived from animals, plants and microorganisms, or a recombinant of natural lysozyme. For example, it can be egg lysozyme, human lysozyme, recombinant human lysozyme, phage lysozyme, etc. The lysozyme in the present disclosure also comprises its medicinal salts, such as hydrochloride, chloride, sulfate or amino acid salt, etc. Egg lysozyme is the main commercial source of lysozyme, and its activity is close to that of human lysozyme. The lysozyme used in many marketed drugs is egg lysozyme. Human lysozyme is widely distributed and abundant in human body. Lysozyme has high safety.

Enteric formulation: the enteric formulation refers to a formulation that does not release or almost does not release drug in the stomach, but can release most or all of the drug in some parts of the intestine after entering the intestine. Human intestines include small intestine and large intestine, in which the small intestine is also divided into duodenum, jejunum and ileum, and the large intestine is also divided into cecum, colon and rectum. Different parts of the digestive tract have different pH values. For example, the pH value in the stomach is about 1-3, the pH value in the small intestine is about 4-7, and the pH value in the large intestine is about 7-8. By selecting pH dependent degradation materials as formulation adjuvants, the formulations that release drugs in specific parts of the digestive tract can be prepared, such as small intestinal enteric formulations or large intestinal enteric formulations. Specifically, it can comprise a duodenal enteric formulation, a jejunal enteric formulation, an ileal enteric formulation, a cecal enteric formulation, a colon enteric formulation or a rectal enteric formulation, etc.

Exemplary preparation methods of a separate formulation of dosmalfate, a separate formulation of lysozyme, and a pharmaceutical composition of dosmalfate and lysozyme are described below.

Raw materials of lysozyme can be commercially available, and has a potency of above 20000 U/mg; raw materials of dosmalfate can be prepared according to the existing technical method.

Dosmalfate tablet: the dosmalfate tablet is obtained by tableting using common tablet adjuvants based on dosmalfate as a raw material.

Lysozyme granules: the granules are prepared by using lysozyme as a raw material, adding an equal amount of starch, evenly mixing, adding an adhesive (water) and then drying.

Lysozyme colon enteric-coated granules: the lysozyme granules are prepared by using lysozyme as a raw material, adding an equal amount of starch, evenly mixing, adding an adhesive (water) to granulation and then drying to obtain the lysozyme granules. The lysozyme colon enteric-coated granules are obtained by coating the prepared lysozyme granules using commercially available colon enteric coatings according to a conventional method.

Dosmalfate-lysozyme compounded capsules: the lysozyme granules are prepared by using lysozyme as a raw material, adding an equal amount of starch, evenly mixing, adding an adhesive (water) to granulation and then drying to obtain the lysozyme granules. The lysozyme colon enteric-coated granules are obtained by coating the prepared lysozyme granules using commercially available colon enteric coatings according to a conventional method. The dosmalfate granules are prepared by using dosmalfate as a raw material, adding common granule adjuvants and granulating. The lysozyme colon enteric-coated granules and the dosmalfate granules are put into common empty capsules in a proper proportion to obtain the dosmalfate-lysozyme compounded capsules.

The present disclosure will be illustrated in detail through animal experiments in the following examples.

The used test animals, reagents and others are commercially available. Relevant testing methods and specific operation manners of related instruments used in tests are all well-known to the skilled in the art. The used dosmalfate and lysozyme colon enteric-coated granules are all provided by Xiangbei Welman Pharmaceutical Co., Ltd.

### Examples

### Therapeutic effect of a combination of dosmalfate and lysozyme on irritable bowel syndrome model animals

In this example, the irritable bowel syndrome model was induced by daily applying colorectal distension as a stimulating factor to neonatal SD rats (Sprague-Dawley rats) from 8 days old to 21 days old. This model animal had visceral hypersensitivity of irritable bowel syndrome and without colonitis and mucosal injury. Colorectal distension stimulation was not given any more from 21 days old to 8 weeks old. A test drug or placebo was administered daily by gavage from 8 weeks old to 3 months old. On the 8 weeks old (before the beginning of the 1-month drug administration period) and about 3 months old (after the end of the 1-month drug administration period), behavioral and neural responses of rats to colorectal distention stimulation were tested by an abdominal wall withdrawal reflex test and an electromyography test.

### 1. Animals and grouping

8-day-old neonatal male SD rats with normal body weight were exposed to a 12:12 hr light-dark cycle. Every 12 neonatal rats and 1 adult female rat were co-bred with rat milk until 25 days old. Then every 4 neonatal rats were fed in the same cage, and they were allowed to eat and drink freely.

The SD rats were randomly divided into a normal control group, a model group, a dosmalfate group, a lysozyme group, a first combination group (a dosage ratio of dosmalfate to lysozyme was 0.05:1), a second combination group (a dosage ratio of dosmalfate to lysozyme was 0.1:1), a third combination group (a dosage ratio of dosmalfate to lysozyme was 10:1), and a fourth combination group (a dosage ratio of dosmalfate to lysozyme was 20:1)., and 12 animals were assigned to each group.

### 2. Modeling

Except for normal control group, colorectal distension (CRD) stimulation was applied to the rats in other groups every day from 8 days old to 21 days old to induce irritable bowel syndrome. The concrete operation method was as follows: a flexible balloon (with a polytetrafluoroethylene catheter; the balloon should be inflated overnight before the experiment so that it has better compliance) made of the fingers of surgical gloves, was smeared with vaseline, inserted from the anus of the rat to the colon and then fixed at the tail distanced away from the anus by 2cm. The balloon was connected with a valve via the catheter, and the valve was additionally connected with a sphygmomanometer. Rats were placed in small plastic cages to restrict their turning and acclimatized for 30 minutes. The balloon was slowly inflated to 60 mm Hg with the sphygmomanometer, and deflated after stimulation for 20 seconds; the stimulation was repeated twice within 1 hour (with an interval of 30 minutes each time).

Rats in normal control group were treated with the method similar to that in other groups, but the balloon was not inserted into the colon, and the rats were gently palpated in the perineal area every day from 8 days old to 21 days old.

When the modeling was completed, only one rat in normal control group died, and the follow-up experimental processes were completed for the rest animals.

### 3. Preparation and administration of drugs

Except for normal control group and model group, test drugs (wetted in 0.5% sodium carboxymethyl cellulose solution) were administrated to the rats in the other groups by gavage daily from 8 weeks old to 3 months old, and the dosage was 8 ml.

Lysozyme group: 100 mg/kg (calculated by lysozyme) of lysozyme colon enteric-coated granules.

Dosmalfate group: 100 mg/kg of dosmalfate.

First combination group: 100 mg/kg (calculated by lysozyme) of lysozyme colon enteric-coated granules, and 5 mg/kg of dosmalfate.

Second combination group: 100 mg/kg (calculated by lysozyme) of lysozyme colon enteric-coated granules, and 10 mg/kg of dosmalfate.

Third combination group: 100 mg/kg (calculated by lysozyme) of lysozyme colon enteric-coated granules, and 1000 mg/kg of dosmalfate.

Fourth combination group: 100 mg/kg (calculated by lysozyme of lysozyme colon enteric-coated granules, and 2000 mg/kg of dosmalfate.

An equal amount of 0.5% sodium carboxymethyl cellulose solution was daily administrated to model group. No drugs were administrated to normal control group.

### 4. Test

The colorectal distension (CRD) model has become a standard tool for assessing visceral sensitivity in animals. Abdominal wall withdrawal reflex and EMG tests are the most commonly used methods to evaluate animal responses after colorectal distension stimulation.

### 4.1 Abdominal withdrawal reflex (AWR) test

The abdominal wall withdrawal reflex test was used to detect behavioral responses of rats to colorectal distension stimulation. This test was performed when rats in respective groups were 8 weeks old (before the start of the 1-month drug administration period) and 3 months old (after the end of the 1-month drug administration period). The rats were fasted overnight before undergoing the abdominal wall withdrawal reflex test, so as to reduce gastrointestinal contents.

During the test, a flexible balloon (with a thin-walled polytetrafluoroethylene catheter; the balloon should be inflated overnight before the experiment so that it has better compliance) made of the fingers of surgical gloves, was smeared with vaseline and inserted from the anus of the rat by 7 cm to the colon and fixed at the caudal point by 2cm distanced away from the anus. The balloon was connected with the valve via the catheter, and the valve was additionally connected with a sphygmomanometer. Rats were placed in small plastic cages to restrict their turning and acclimatized for 30 minutes. The balloons were inflated with the sphygmomanometer to be tested under 20, 40 and 60 mm Hg pressures with the sphygmomanometer. During each test, the pressure was maintained for 20 seconds, and the abdominal wall withdrawal reflex score of the rats during the 20 seconds of pressure maintenance was recorded by a blinded observer. The test was repeated 3 times under each pressure condition, and the average value of the 3 tests was taken as a result. Rats were allowed to rest for 10 minutes between every two tests and also between different stresses.

The abdominal wall withdrawal reflex (AWR) has a scoring rule as following: 0=no behavioral response; 1=transient movement of a head, followed by immobility; 2=abdominal muscle contraction; 3=lifting the abdomen off the platform of cage; 4=pelvic structure arched.

### 4.2 Electromyography (EMG) test

The visceral hypersensitivity (neural response) of rats to colorectal distension was quantified by measuring the electromyographic activity of external oblique abdominal muscles. When the rats in each group were 3 months old (after the end of the 1-month drug administration period), they were subjected to abdominal wall withdrawal reflex test followed by EMG test after abdominal wall withdrawal reflex test.

On the second day when 3-month-old rats in each group were subjected to abdominal wall withdrawal reflex test, an appropriate amount of pentobarbital solution (50 mg/ml) was injected into the abdominal cavities of the rats, and the rats were fully anesthetized. The hairs on the lower abdomen and the back of the neck were cut, a needle electrode (tungsten microelectrode) was implanted into the left side of the abdomen of the rat, fixed in the muscle layer of the external oblique abdominal muscle, and the electrode wire was crossed through the subcutaneous of the left side of the rat to a small incision at the rear neck and out of the small incision. The wounds on the abdomen and neck of the rat were sutured and the rats were returned to the cage. On the day 3 after surgery, the rats were fasted overnight to reduce gastrointestinal contents. On the day 4 after surgery, electromyography test was started. The colorectal distention stimulation was performed with the method identical to that in abdominal wall withdrawal reflex test, and the discharge activity of the external oblique abdominal muscle of the rat under the stimulation of each colorectal distention pressure (20, 40 and 60 mm Hg) was recorded by using a physiological signal acquisition and processing system. The pressure was maintained for 20 seconds each time, each pressure was tested 3 times, and the average value of the 3 tests was taken as the result. Rats were allowed to rest for 10 minutes between each test and also between different stresses.

The EMG test results are represented by the area under the curve (AUC) during the 30-second period after the onset of colorectal distention, and each datum is normalized relative to the average baseline amplitude (set to 100%).

### 5. Test results and evaluation

### Abdominal withdrawal reflex and EMG tests

The higher score of the abdominal wall withdrawal reflex indicates the stronger behavior response of the rat to colorectal distension stimulation and further indicates the higher visceral sensitivity, thus showing the more severe irritable bowel syndrome.

The larger AUC value (normalized relative to the average baseline amplitude) of EMG indicates the stronger neural response of the rat to colorectal distension stimulation, and further indicates higher visceral sensitivity, thus showing the more severe irritable bowel syndrome. The statistical results of this test are listed in Tables 1-3.

**Table 1: Abdominal withdrawal reflex test score results of animals in each group on 8 weeks old**

| Groups | Numbers of rats | Abdominal withdrawal reflex scores of colorectal distension at different pressures (mean ± SD ) | | |
|---|---|---|---|---|
| | | 20 mm Hg | 40 mm Hg | 60 mm Hg |
| Normal control group | 11 | 0.64 ± 0.10 | 1.21 ± 0.22 | 2.33 ± 0.30 |
| Model group | 12 | 2.39 ± 0.53^{∗∗} | 2.67 ± 0.40^{∗∗} | 3.58 ± 0.41^{∗∗} |
| Dosmalfate group | 12 | 2.31 ± 0.41^{∗∗} | 2.50 ± 0.46^{∗∗} | 3.56 ± 0.48^{∗∗} |
| Lysozyme group | 12 | 2.14 ± 0.30^{∗∗} | 2.58 ± 0.47^{∗∗} | 3.61 ± 0.19^{∗∗} |
| First combination group | 12 | 2.33 ± 0.53^{∗∗} | 2.42 ± 0.35^{∗∗} | 3.42 ± 0.38^{∗∗} |
| Second combination group | 12 | 2.28 ± 0.42^{∗∗} | 2.50 ± 0.30^{∗∗} | 3.50 ± 0.30^{∗∗} |
| Third combination group | 12 | 2.31 ± 0.54^{∗∗} | 2.56 ± 0.26^{∗∗} | 3.64 ± 0.27^{∗∗} |
| Fourth combination group | 12 | 2.25 ± 0.38^{∗∗} | 2.42 ± 0.21^{∗∗} | 3.42 ± 0.35^{∗∗} |

| | | | | |
|---|---|---|---|---|
| ^{∗∗} represents that there is significant difference compared with normal control group under the corresponding pressure (P < 0.01). | | | | |

**Table 2: Abdominal withdrawal reflex test score results of animals in each group at 3 months old**

| Groups | Numbers of rats | Abdominal withdrawal reflex scores of colorectal distension at different pressures (mean ± SD ) | | |
|---|---|---|---|---|
| | | 20 mm Hg | 40 mm Hg | 60 mm Hg |
| Normal control group | 11 | 0.70 ± 0.23 | 1.12 ± 0.27 | 2.36 ± 0.32 |
| Model group | 12 | 2.45 ± 0.56^{∗∗} | 2.72 ± 0.45^{∗∗} | 3.67 ± 0.43^{∗∗} |
| Dosmalfate group | 12 | 2.25 ± 0.45 | 2.56 ± 0.30 | 3.64 ± 0.30 |
| Lysozyme group | 12 | 2.11 ± 0.33 | 2.69 ± 0.41 | 3.56 ± 0.36 |
| First combination group | 12 | 2.08 ± 0.32 | 2.39 ± 0.37 | 3.44 ± 0.43 |
| Second combination group | 12 | 1.06±0.24^{##&&} | 1.56±0.26 ^{##&&} | 2.39±0.34^{##&&} |
| Third combination group | 12 | 1.64±0.27^{##&} | 1.72±0.31 ^{##*&&*} | 2.42±0.38 ^{##*&&*} |
| Fourth combination group | 12 | 2.11 ± 0.36 | 2.47 ± 0.36 | 2.89±0.46^{##*&&*} |

| | | | | |
|---|---|---|---|---|
| ^{∗∗} represents that there is significant difference compared with normal control group under the corresponding pressure (P < 0.01), ## represents that there is significant difference compared with model group under the corresponding pressure (P < 0.01), && represents that there is significant difference compared with lysozyme group under the corresponding pressure (P < 0.01), & represents that there is difference compared with lysozyme group under the corresponding pressure (P < 0.05). | | | | |

**Table 3: EMG test score results of animals in each group at 3 months old**

| Groups | Numbers of rats | AUC (%) of EMG of colorectal distension at different pressures (mean ± SD ) | | |
|---|---|---|---|---|
| | | 20mm Hg | 40mm Hg | 60mm Hg |
| Normal control group | 11 | 141 ± 35 | 184 ± 17 | 273 ± 39 |
| Model group | 12 | 303 ± 37^{∗∗} | 437 ± 104^{∗∗} | 637 ± 104^{∗∗} |
| Dosmalfate group | 12 | 332 ± 39 | 445 ± 99 | 644 ± 117 |
| Lysozyme group | 12 | 303 ± 36 | 394 ± 60 | 601 ± 60 |
| First combination group | 12 | 286 ± 28 | 405 ± 80 | 580 ± 41 |
| Second combination group | 12 | 245 ± 27^{##*&&*} | 268 ± 42 ^{##*&&*} | 334 ± 37^{##*&&*} |
| Third combination group | 12 | 229 ± 46^{##*&&*} | 299 ± 30^{##*&*} | 356 ± 38^{##*&&*} |
| Fourth combination group | 12 | 272 ± 48 | 310 ± 67^{##*&*} | 421 ± 34^{##*&&*} |

| | | | | |
|---|---|---|---|---|
| ^{∗∗} represents that there is significant difference compared with normal control group under the corresponding pressure (P < 0.01), ## represents that there is significant difference compared with model group under the corresponding pressure (P < 0.01), && represents that there is significant difference compared with lysozyme group under the corresponding pressure (P < 0.01), & represents that there is difference compared with lysozyme group under the corresponding pressure (P < 0.05). | | | | |

In this test, colorectal distension stimulation is continuously applied to neonatal rats to induce generation of irritable bowel syndrome model, resulting in high visceral sensitivity. Colorectal distension (CRD) is a widely used and repeatable visceral sensitivity evaluation method in preclinical and clinical researches. As a harmful stimulation to the intestine, a visceral pain response can be caused by applying colorectal distention stimulation to subjects or tested animals with visceral hypersensitivity. This mechanical distention model can better simulate symptoms occurring in patients with irritable bowel syndrome, such as abdominal pain and abdominal discomfort, and this visceral stimulation form is easy to control and has good repeatability. Colorectal distension is often used to evaluate visceral sensitivity in rodents (such as rats and mice). The colorectal distension model has become a standard tool to evaluate visceral sensitivity in rodents.

It can be seen from the results of abdominal wall withdrawal reflex and electromyography tests that the irritable bowel syndrome animal model was successfully established. The animal model has visceral hypersensitivity, which can be compared with various types of irritable bowel syndromes in humans. Compared with normal control group, the behavioral responses and neural responses of animals in model group to colorectal distention stimulation are significantly strengthened under each test pressure. It can be seen from data in Tables 1-3 that compared with normal control group, the abdominal wall withdrawal reflex score of the model group is increased significantly (P < 0.01), and the AUC value of the area under the curve of EMG test is also increased significantly (P < 0.01), indicating that the animals in model group maintained the visceral hypersensitivity state of irritable bowel syndrome at least from 8 weeks old to 3 months old.

The following conclusions can be drawn from the test results:
1. After administration, there is no significant difference in numeral values of the abdominal wall withdrawal reflex and EMG test result between lysozyme group and model group (P>0.05), indicating that the alone use of lysozyme basically has no curative effect on the treatment of irritable bowel syndrome. Dosemalate group is similar to lysozyme group.
2. After administration, compared with model group, numeral values of the abdominal wall withdrawal reflex and EMG test result in first combination group tend to reduce, but there is no statistical difference between them, indicating that the ratio of dosemalate to lysozyme in the combination can affect the curative effect on irritable bowel syndrome, so combination of dosemalate and lysozyme should reach a certain ratio in order to produce a better synergistic effect.
3. After administration, compared with model group and lysozyme group, numeral values of the abdominal wall withdrawal reflex and EMG test result in second combination group, third combination group and fourth combination group are significantly reduced (P<0.01), indicating that the combination of dosemalate and lysozyme functions that the two components do not have when they are used alone, and plays a synergistic effect, and can be used for treatment of irritable bowel syndrome.

In this test, on the day when EMG test was completed, the rats were sacrificed, and tissues such as brain, heart, liver, spleen, lung, kidney and digestive tract (including colon) etc. were subjected to histopathological examinations. Compared with normal control group and model group, there were no obvious pathological changes in the rats in first combination group, second combination group, third combination group and fourth combination group, indicating that the combination of the present disclosure has small toxic and side effects after administration, as well as high safety. In addition, in the present disclosure, there are no typical inflammations, mucosal damages or other abnormalities in colonic histopathological examination, thus the association of inflammation and mucosal damage with the visceral hypersensitivity of test animals is excluded.

The present disclosure has been described in detail above with general descriptions, specific embodiments and the like. On the basis of the present disclosure, the skilled in the art can make reasonable modifications or improvements to the present disclosure, and these modifications or improvements made without departing from the spirit of the present disclosure belong to the content of the present disclosure.

## Claims

1. Use of a drug combination in the preparation of a medicament for preventing or treating irritable bowel syndrome, wherein the drug combination comprising dosmalfate and lysozyme.

2. The use according to claim 1, wherein a mass ratio of dosmalfate to lysozyme in the drug combination is (0.05-100):1, and does not comprise 0.05:1.

3. The use according to claim 1, wherein the mass ratio of dosmalfate to lysozyme in the drug combination is (0.1-10): 1.

4. A pharmaceutical composition for preventing or treating irritable bowel syndrome, wherein the pharmaceutical composition comprising dosmalfate and lysozyme.

5. The pharmaceutical composition according to claim 4, wherein a mass ratio of dosmalfate to lysozyme in the pharmaceutical composition is (0.05-100):1, and does not comprise 0.05:1.

6. The pharmaceutical composition according to claim 5, wherein the mass ratio of dosmalfate to lysozyme in the pharmaceutical composition is (0.1-10): 1.

7. The pharmaceutical composition according to any one of claims 4-6, wherein the pharmaceutical composition is an oral formulation, preferably, the oral formulation is any one selected from an oral normal-release formulation, an oral sustained-release formulation and an oral controlled-release formulation, more preferably, the oral formulation is any one selected from a tablet, a capsule, a granule, powder, a pill, syrup, an oral solution, an oral suspension and an oral emulsion.

8. The pharmaceutical composition according to any one of claims 4-6, wherein the pharmaceutical composition also comprises pharmaceutically acceptable adjuvants, preferably, the pharmaceutically acceptable adjuvants are at least one selected from fillers, adhesives, disintegrating agents, lubricants, solubilizers, flavoring agents, colorants, masking agents, pH regulators, suspending agents, thickeners, preservatives, stabilizers, antioxidants, wetting agents, surfactants, suspending agents, absorption enhancers and coating materials.

9. A method for preparing the pharmaceutical composition according to any one of claims 4-8, comprising the following step: mixing dosmalfate with lysozyme.

10. The method for preparing the pharmaceutical composition according to claim 8, comprising the following steps: preparing a dosmalfate formulation subunit and a lysozyme formulation subunit with dosmalfate and lysozyme as well as the pharmaceutically acceptable adjuvants respectively, and mixing the two formulation subunits to obtain the pharmaceutical composition; preferably, the dosmalfate formulation subunit being in an enteric dosage form or a gastric dosage form, and the lysozyme formulation subunit being in the enteric dosage form.
